(19) Europäisches Patentamt
European Patent Office
Office européen des brevets

(11) **EP 1 390 023 B1**

(12) **EUROPÄISCHE PATENTSCHRIFT**

(45) Veröffentlichungstag und Bekanntmachung des Hinweises auf die Patenterteilung:
**14.05.2008 Patentblatt 2008/20**

(21) Anmeldenummer: **02716816.0**

(22) Anmeldetag: **28.02.2002**

(51) Int Cl.:
*A61K 31/137* (2006.01)     *A61K 31/18* (2006.01)
*A61K 31/428* (2006.01)     *A61K 31/54* (2006.01)
*A61P 13/00* (2006.01)     *A61P 29/00* (2006.01)
*C07D 489/04* (2006.01)

(86) Internationale Anmeldenummer:
**PCT/EP2002/002169**

(87) Internationale Veröffentlichungsnummer:
**WO 2002/067916 (06.09.2002 Gazette 2002/36)**

(54) **PHARMAZEUTISCHE SALZE VON 1-PHENYL-3-DIMETHYLAMINO-PROPAN-VERBINDUNGEN**

PHARMACEUTICAL SALTS OF 1-PHENYL-3-DIMETHYLAMINO-PROPANE COMPOUNDS

SELS PHARMACEUTIQUES DES COMPOSÉS 1-PHENYL-3-DIMETHYLAMINO-PROPANIQUES

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **28.02.2001 DE 10109763**

(43) Veröffentlichungstag der Anmeldung:
**25.02.2004 Patentblatt 2004/09**

(73) Patentinhaber: **Grünenthal GmbH**
**52078 Aachen (DE)**

(72) Erfinder:
• **BARTHOLOMÄUS, Johannes**
**52080 Aachen (DE)**

• **KUGELMANN, Heinrich**
**52068 Aachen (DE)**

(74) Vertreter: **Kutzenberger, Helga et al**
**Kutzenberger & Wolff,**
**Theodor-Heuss-Ring 23**
**50668 Köln (DE)**

(56) Entgegenhaltungen:
**EP-A- 0 061 654     EP-A- 1 136 487**
**EP-A- 1 270 580     WO-A-00/12067**
**WO-A-01/15667     WO-A-02/43715**
**DE-A1- 4 426 245     US-A- 4 362 730**
**US-A1- 2001 041 738**

EP 1 390 023 B1

**Beschreibung**

Die vorliegende Erfindung betrifft pharmazeutische Salze aus einem Wirkstoff und wenigstens einem Zuckerersatzstoff, Arzneimittel enthaltend diese Salze sowie die Verwendung dieser Salze zur Herstellung von Arzneimitteln.

Eine Vielzahl von pharmazeutischen Wirkstoffen mit ausgezeichneter Wirksamkeit führt bei oraler Gabe zu einem stark bitteren, häufig ekelerregenden Geschmacksempfinden beim Patienten. Bei manchen Patienten resultiert aus diesem negativen Geschmackseriebnis eine mangelnde Einhaltung der Dosierungsvorschrift sowie eine mangelnde Akzeptanz der entsprechenden Arzneimittel, die einen solchen Wirkstoff bereits bei der Einnahme freisetzen.

Die Formulierung von pharmazeutischen Wirkstoffen mit sehr guter Wasserlöslichkeit zu Arzneimitteln bereitet in der pharmazeutischen Praxis häufig Probleme, So ist die Herstellung von Arzneiformen mit kontrollierter Freisetzung aufgrund der sehr guten Wasserlöslichkeit von Wirkstoffzalzen oftmals erschwert. Eine Retardierung dieser Wirkstoffe kann zwar beispielsweise durch das Überziehen der Arzneiformen mit retardierenden Filmüberzügen erreicht werden. Diese Art der Retardierung ist aber mit einem relativ hohen Aufwand verbunden, da retardierende Filmüberzüge aus wäßrigen Überzugssystemen für sehr gut wasserlösliche Wirkstoffe häufig nur eine unzureichende Diffusionsbarriere darstellen. Die Herstellung dieser retardierten Wirkstoff-Zubereitungen erfordert daher relativ aufwendige Überzugsverfahren mit mehrschichtigen Filmen. Sofern solche retardierenden Überzüge aus organischen Lösungsmitteln aufgebracht werden, verteuert die damit verbundene Umwelt- und Lösungsmittelrückstandproblematik zusätzlich die Herstellung entsprechender Zubereitungen. Aufgabe der vorliegenden Erfindung war es daher, pharmazeutische Verbindungen von Wirkstoffen zur Verfügung zu stellen, die keinen bitteren Geschmack aufweisen. Vorzugsweise sollen sich die entsprechenden Wirkstoffverbindungen einfacher formulieren und effektiver retardieren lassen.

Erfindungsgemäß wird diese Aufgabe durch die Bereitstellung pharmazeutischer, d.h. physiologisch verträglicher Salze aus einem pharmazeutischem Wirkstoff und wenigstens einem Zuckerersatzstoff gelöst, wobei der Wirkstoff eine salzbildende Verbindung ist ausgewählt aus der Gruppe

(1RS,2RS)-3-(3-Dimethylamino-1-hydroxy-1,2-dimethyl-propyl)phenol,
(-)-(1R,2R)-3-(3-Dimethylamino-1-ethyl-2-methyl-propyl)-phenol,
(+)-(1S,2S)-3-(3-Dimethylamino-1-ethyl-2-methyl-propyl)-phenol,
(2RS,3RS)-1-Dimethylamino-3-(3-methoxy-phenyl)-2-methyl-pentan-3-ol,
(-)-(1S,2S)-3-(3-Dimethylamino-1-ethyl-1-fluoro-2-methyl-propyl)-phenol,
(+)-(1R,2R)-3-(3-Dimethylamino-1-hydroxy-1,2-dimethyl-propyl)-phenol,
(+)-(2R,3R)-1-Dimethylamino-3-(3-methoxy-phenyl)-methyl-pentan-3-ol und
(-)-(2S,3S)-1-Dimethylamino-3-(3-methoxy-phenyl)-2-methyl-pentan-3-ol.

Gegenstand der vorliegenden Erfindung sind daher pharmazeutische Salze aus einem pharmazeutischem Wirkstoff und wenigstens einem Zuckerersatzstoff, wobei der Wirkstoff eine salzbildende Verbindung ist ausgewählt aus der Gruppe

(1RS,2RS)-3-(3-Dimethylamino-1-hydroxy-1,2-dimethyl-propyl)phenol,
(-)-(1R,2R)-3-(3-Dimethylamino-1-ethyl-2-methyl-propyl)-phenol,
(+)-(1S,2S)-3-(3-Dimethylamino-1-ethyl-2-methyl-propyl)-phenol,
(2RS,3RS)-1-Dimethylamino-3-(3-methoxy-phenyl)-2-methyl-pentan-3-ol,
(-)-(1S,2S)-3-(3-Dimethylamino-1-ethyl-1-fluoro-2-methyl-propyl)-phenol,
(+)-(1R,2R)-3-(3-Dimethylamino-1-hydroxy-1,2-dimethyl-propyl)-phenol,
(+)-(2R,3R)-1-Dimethylamino-3-(3-methoxy-phenyl)-methyl-pentan-3-ol und
(-)-(2S,3S)-1-Dimethylamino-3-(3-methoxy-phenyl)-2-methyl-pentan-3-ol.

In einer bevorzugten Ausführungsform der vorliegenden Erfindung beträgt die Löslichkeit der erfindungsgemäßen pharmazeutischen Salze in Wasser ≤ 250 mg/ml Wasser, bevorzugt ≤ 200 mg/ml, besonders bevorzugt ≤ 150 mg/ml, ganz besonders bevorzugt ≤ 100 mg/ml. Dies zeigt sich insbesondere auch dadurch, daß die Wasserlöslichkeit der erfindungsgemäßen pharmazeutischen Salze gegenüber der Wasserlöslichkeit des best wasserlöslichen Salzes des entsprechenden Wirkstoffs gemäß Pharmazeutische Stoffliste, 12. Auflage ABDATA Pharma-Daten-Service, 65735 Eschborn/Taunus, vorzugsweise gegenüber dem entsprechenden Hydrochlorid um mindestens 50 %, vorzugsweise um mindestens 65 %, besonders bevorzugt um mindestens 75 %, ganz besonders bevorzugt um mindestens 85 % verringert ist.

Als Zuckerersatzstoffe kommen erfindungsgemäß alle Zuckerersatzstoffe in Frage, die unter Ausbildung einer wenigstens einfach negativ geladenen Form mit dem jeweiligen pharmazeutischen Wirkstoff ein Salz bilden können. Erfindungsgemäß werden auch pharmazeutische Salze umfaßt, in denen der pharmazeutische Wirkstoff zwei oder mehr verschiedene Zuckerersatzstoffe als Salzpartner aufweist. Bevorzugt enthalten die erfindungsgemäßen pharmazeutischen Salze als salzbildende Zuckerersatzstoffe Saccharin, Cyclamat oder Acesulfam, besonders bevorzugt Saccharin.

Als Wirkstoffe kommen erfindungsgemäß alle pharmazeutischen Wirkstoffe der vorstehend genannten Gruppe in Frage, die unter Ausbildung einer wenigstens einfach positiv geladenen Form mit dem/den jeweiligen Zuckerersatzstoff (en) In anionischer Form ein Salz bilden können.

Die Herstellung der erfindungsgemäßen pharmazeutischen Salze kann nach üblichen, dem Fachmann bekannten Methoden erfolgen. Vorzugsweise werden zur Herstellung der erfindungsgemäßen pharmazeutischen Salze jeweils separat voneinander wenigstens ein Salz des jeweiligen Wirkstoffes und wenigstens ein Salz des jeweiligen. Zuckerersatzstoffs in einer möglichst geringen Menge eines Lösungsmittels oder Lösungsmittelgemisches ggf. unter Erwärmen gelöst.

Anschließend werden beide Lösungen zusammengegeben, gegebenenfalls vermischt und gegebenenfalls gekühlt. Sofern aus der ggf. gekühlten Lösung das erfindungsgemäße pharmazeutische Salz aus dem Wirkstoff und dem Zuckerersatzstoff zumindest teilweise ausfällt, wird dieses nach üblichen Methoden, vorzugsweise durch Saugfiltration abgetrennt. Das abgetrennte pharmazeutische Salz wird dann, sofern erforderlich, nach üblichen, dem Fachmann bekannten Methoden, beispielsweise durch Umkristallisation, Waschen oder durch Rühren in einem geeigneten Lösungsmittel gereinigt.

Sofern das pharmazeutische Salz noch nicht vollständig ausgefallen ist, wird die verbleibende Lösung vorzugsweise am Rotationsverdampfer vollständig eingeengt und das erfindungsgemäße pharmazeutische Salz aus dem Rückstand nach üblichen, dem Fachmann bekannten Methoden extrahiert und wie vorstehend beschrieben gereinigt.

Das jeweils zur Herstellung geeignete Lösungsmittel oder Lösungsmittelgemisch und die geeigneten Umsetzungsbedingungen, wie beispielsweise Temperatur oder Umsetzungsdauer, können vom Fachmann mit Hilfe von einfachen Vorversuchen ermittelt werden.

Sofern sowohl das Wirkstoffsalz als auch das Salz des Zuckerersatzstoffs eine ausreichende Löslichkeit in Wasser aufweisen, wird als Lösungsmittel bevorzugt Wasser verwendet. Als Salz, des jeweiligen Wirkstoffes wird bevorzugt dessen Hydrochlorid, Hydrobromid, Phosphat, Hydrogenphosphat, Hydrogensulfat, Sulfat, Nitrat oder Metilsulfat eingesetzt. Als Salz des jeweiligen Zuckerersatzstoffes wird bevorzugt dessen Natrium-, Kalium-, Kalzium oder Ammoniumsalz eingesetzt.

Selbstverständlich ist auch möglich den jeweiligen Wirkstoff an sich mit der freien Säure eines Zuckerersatzstoffes in einem geeigneten Umsetzungsmedium miteinander umzusetzen und das so erhaltene pharmazeutische Salz nach üblichen, dem Fachmann bekannten Methoden zu isolieren und ggf. zu reinigen.

Ein weiterer Gegenstand der vorliegenden Erfindung sind Arzneimittel enthaltend wenigstens ein erfindungsgemäßes pharmazeutisches Salz und gegebenenfalls physiologisch verträgliche Hilfsstoffe. Die entsprechenden Arzneimittel können für die Behandlung der für die jeweiligen Wirkstoffe bekannten Indikationen verwendet werden.

Vorzugsweise werden erfindungsgemäße Arzneimittel zur Bekämpfung von Schmerzen eingesetzt, die wenigstens ein erfindungsgemäßes pharmazeutisches Salz aufweisen. Vorzugsweise enthalten die erfindungsgemäßen Arzneimittel als pharmazeutische Salze dieser Wirkstoffe die entsprechenden Saccharinate.

Zur Behandlung von Harninkontinenz werden vorzugsweise erfindungsgemäße Arzneimittel eingesetzt, die wenigstens ein erfindungsgemäßes pharmazeutisches Salz aufweisen. Vorzugsweise enthalten die erfindungsgemäßen Arzneimittel als pharmazeutische Salze dieser Wirkstoffe die entsprechenden Saccharinate.

Die erfindungsgemäßen Arzneimittel können In fester, halbfester oder flüssiger Form vorliegen. Vorzugsweise eignen sich die erfindungsgemäßen Arzneimittel zur oralen Applikation.

In einer bevorzugten Ausführungsform liegt das erfindungsgemäße Arzneimittel als Gel, Kaugummi, Saft, Spray, Tablette, Kautablatte, Dragee. Pulver ggf. in Kapseln abgefüllt, leicht rekonstituierbaren Trockenzubereitungen, vorzugsweise als Gel, als wäßriger oder öliger Saft, als Sublingualspray, Tabletten oder Kautabletten formuliert vor.

Ebenfalls bevorzugt kann das erfindungsgemäße Arzneimittel auch in multipartikulärer Form, vorzugsweise in Form von Milkrotabletten, Mikrokapseln, Granulaten, Wirkstoffkristallen oder Pellets, besonders bevorzugt in Form von Mikrotablatten, Granulaten oder Pellets, ggf. in Kapseln abgefüllt oder zu Tabletten verpreßt, formuliert vorliegen.

Sofern das erfindungsgemäße Arzneimittel in Form von Granulaten oder Pellets vorliegt, können diese bevorzugt eine Größe im Bereich von 0,1 bis 3 mm, besonders bevorzugt Im Bereich von 0,5 bis 2 mm aufweisen.

Liegt das erfindungsgemäße Arzneimittel in Form von Mikrotabletten vor, können diese bevorzugt einen Durchmesser im Bereich von 0,5 bis 5 mm, besonders bevorzugt im Bereich von 1 bis 3 mm und ganz besonders bevorzugt im Bereich von 1 bis 2 mm aufweisen.

Liegt das erfindungsgemäße Arzneimittel in Form von Wirkstoffkristallen, Mikropartikeln, Mikropellets oder Mikrokapseln vor, so können diese bevorzugt einen Durchmesser im Bereich von 10 $\mu$m bis 1 mm, besonders bevorzugt im Bereich von 15 $\mu$m bis 0,5 mm und ganz besonders bevorzugt im Bereich von 30 $\mu$m bis 200 $\mu$m aufweisen.

Die erfindungsgemäßen Arzneimittel können außerdem je nach Ausführungsform als weitere Bestandteile die üblichen, dem Fachmann bekannten physiologisch verträglichen Hilfsstoffe enthalten.

Sofern die erfindungsgemäßen Arzneimittel in Form von Tabletten oder Mikrotabletten vorliegen, können diese als physiologisch verträglichen Hilfsstoffe vorzugsweise mikrokristalline Cellulose, Celluloseether, Lactose, Stärke, Stärkederivate, Zuckeralkohole, Calciumhydrogenphosphat sowie die üblichen, dem Fachmann bekannten Bindemittel, Fließregulationsmittel, Gleitmittel und/oder Sprengmittel enthalten.

Sofern die erfindungsgemäßen Arzneimittel in Form von Gelen oder Kaugummis vorliegen, können diese als physiologisch verträgliche Hilfsstoffe vorzugsweise Methylparaben, Propylparaben, Xylitol und/oder Xanthangummi enthalten.

Liegen die erfindungsgemäßen Arzneimittel in Form von Pellets, Granulaten oder Mikropellets vor, können diese als physiologisch verträgliche Hilfsstoffe bevorzugt mikrokristalline Cellulose, Celluloseether, Lactose, Stärke und Stärkederivate, Zuckeralkohole, Calciumhydrogenphosphat, Fettalkohole, Ester des Glycerins oder Fettsäureester enthalten.

Liegen die erfindungsgemäßen Arzneimittel in Form von Mikrokapseln oder Mikropartikeln vor, so können diese je nach Art des zu Ihrer Herstellung eingesetzten Verfahrens die üblichen, dem Fachmann bekannten physiologisch verträglichen Hilfsstoffe enthalten.

Die Herstellung der erfindungsgemäßen Arzneimittel kann nach üblichen, dem Fachmann bekannten Methoden erFolgen.

Sofern die erfindungsgemäßen Arzneimittel in Form von Tabletten vorliegen, werden vorzugsweise das erfindungsgemäße pharmazeutische Salz und ggf, die physiologisch verträglichen Hilfsstoffe vorzugsweise homogen miteinander vermischt, mittels Feucht-, Trocken-, oder Schmelzgranulation zu Granulaten verabeitet und zu Tabletten verpreßt oder durch direkte Tablettierung des pharmazeutischen Salzes mit weitereren Hilfsstoffen hergestellt. Desweiteren können die Tabletten bevorzugt durch Verpressen ggf. überzogener Pellets, Wirkstoffkristalle, Mikropartikel oder Mikrokapseln hergestellt werden.

Die erfindungsgemäßen Arzneimittel in Form von Pellets können vorzugsweise durch Mischen des pharmazeutischen Salzes und physiologisch verträglicher Hilfsstoffe, Extrusion und Spheronisation, durch Aufbaupelletisierung oder durch Direktpelletisierung in einem hochtourigen Mischer oder in der Rotorwirbelschicht hergestellt werden. Besonders bevorzugt ist die Herstellung der Pellets durch Extrusion feuchter Massen und anschließender Spheronisation.

Die Herstellung von Mikrokapseln erfolgt nach üblichen Mikroverkapselungsverfahren, wie z.B. durch Sprühtrocknung, Sprüherstarrung oder Koazervation.

Die erfindungsgemäßen Arzneimittel in halbfesfer Form, wie beispielsweise Gels oder Kaugummis eignen sich bevorzugt zur Applikation des erfindungsgemäßen pharmazeutischen Salzes über die Mundschleimhaut, die erfindungsgemäßen Arzneimittel in fester oder flüssiger Form, wie beispielsweise ölige oder wäßrige Säfte, Tabletten oder multipartikuläre Formen eignen sich bevorzugt zur Applikation des erfindungsgemäßen pharmazeutischen Salzes über den Magentrakt.

Sofern aus dem erfindungsgemäßen Arzneimittel in fester Form die Wirkstoffaufnahme erst über den Darmtrakt vorgesehen ist, müssen sie wenigstens einen magensaftresistenten Überzug aufweisen. Durch diesen magensaftresistenten Überzug wird erreicht, daß sie den Magentrakt unaufgelöst passieren und das pharmazeutische Salz erst im Darmtrakt zur Freisetzung gelangt. Vorzugsweise löst sich der magensaftresistente Überzug bei einem pH-Wert zwischen 5 und 7,5 auf.

Das erfindungsgemäße Arzneimittel kann das erfindungsgemäße pharmazeutische Salz auch teilweise oder vollständig in retardierter Form aufweisen.

Die Retardierung der Wirkstoffabgabe basiert vorzugsweise auf dem Anbringen eines retardierenden Oberzugs, auf der Einbettung in eine retardierende Matrix, der Anbindung an ein Ionenaustauscherharz oder auf einer Kombination dieser vorstehend genannten Retardierungsmethoden.

Bevorzugt basiert der retardierende Überzug auf einem wasserunlöslichen, gegebenenfalls modifizierten natürlichen oder synthetischen Polymeren oder auf einem natürlichen, halbsynthetischen oder synthetischen Wachs oder Fett oder Fettalkohol oder einem Gemisch aus wenigstens zwei dieser vorstehend genannten Komponenten.

Als wasserunlösliche Polymere werden zur Herstellung eines retardierenden Überzugs vorzugsweise Poly(meth)acrylate, besonders bevorzugt Poly($C_{1-4}$)-alkyl(meth)acrylate, Poly($C_{1-4}$)dialkylamino-($C_{1-4}$)-alkyl(meth)acrylate und/oder deren Copolymere, ganz besonders bevorzugt Ethylacrylat/Methylmethacrylat-Copolymere mit einem molaren Verhätlnis der Monomeren von 2 : 1, Ethylacrylat/Methylmethacrylat/Trimethylammoniumethylmethacrylatchlorid-Copolymere mit einem molaren Verhältnis der Monomeren von 1: 2 : 0,1, Ethylacrylat/Methylmethacrylat/Trimethylammoniumethylmethacrylatchlorid-Copolymere mit einem molaren Verhältnis der Monomeren von 1 : 2 : 0,2 oder ein Gemisch aus wenigstens zwei dieser vorstehend genannten Polymeren als Überzugsmaterial eingesetzt.

Diese Überzugsmateriallen sind als 30 Gew.%-ige wäßrige Latexdispersionen unter den Bezeichnungen Eudragit RS30D®, Eudragit NE30D® bzw. Eudragit RL30D® am Markt erhältlich und werden als solche auch als Überzugsmaterial bevorzugt eingesetzt.

Ebenfalls bevorzugt können als wasserunlösliche Polymere zur Herstellung des retardierenden Überzugs für die erfindungsgemäßen Arzneimittel Polyvinylacetate ggf. in Kombination mit weiteren Hilfstoffen eingesetzt werden. Diese sind als wäßrige Dispersion enthaltend 27 Gew.-% Polyvinylacetat, 2,5 Gew.-% Povidon und 0,3 Gew.-% Natriumlaurylsulfat (Kollicoat SR 30 D®) am Markt erhältlich.

In einer weiteren bevorzugten Ausführungsform basieren die retardierenden Überzüge der erfindungsgemäßen Arzneimittel auf wasserunlöslichen Cellulosederivaten, vorzugsweise Alkylcellulosen, wie z.B. Ethylcellulose, oder auf Celluloseestern, wie z.B. Celluloseacetat als Überzugsmaterial. Die Überzüge aus Ethylcellulose oder Celluloseacetat werden bevorzugt aus wäßriger Pseudolatexdispersion aufgebracht. Wäßrige Ethylcellulose-Pseudolatexdispersionen werden als 30 Gew.%-Ige Dispersionen (Aquacoat®) oder als 25 Gew.%-ige Dispersionen (Surelease®) am Markt geführt und werden als solche auch bevorzugt als Überzugsmaterial eingesetzt

Als natürliche, halbsynthetische oder synthetische Wachse, Fette bzw. Fettalkohole kann der retardierende Überzug in dem erfindungsgemäßen Arzneimittel, vorzugsweise Carnaubawachs, Bienenwachs, Glycerinmonostearat, Glycerinmonobehenat (Compritol ATO888®), Glyceringtripalmitostearat (Precirol ATO5®), mikrokristallines Wachs, Cetylalkohol, Cetylstearylalkohol oder ein Gemisch aus wenigstens zwei dieser Komponenten aufweisen.

Sofern der retardierende Überzug auf einem wasserunlöslichen, gegebenenfalls modifizierten natürlichen und/ oder synthetischen Polymeren basiert, kann die Überzugsdispersion oder Lösung neben dem entsprechendem Polymer einen üblichen, dem Fachmann bekannten, physiologisch verträglichen Weichmacher aufweisen, um die notwendige Mindestfilmtemperatur zu senken.

Geeignete Weichmacher sind beispielsweise lipophile Diester aus einer aliphatischen oder aromatischen Dicarbonsäure mit $C_6$-$C_{40}$ und einem aliphatischen Alkohol mit $C_1$-$C_8$, wie z.B. Dibutylphthalat, Diethylphthalat, Dibutylsebacat oder Diethylsebacat, hydrophile oder lipophile Ester der Zitronensäure, wie z.B. Triethylcitrat, Tributylcitrat, Acetyltributylcitrat oder Acetyltriethylcitrat, Polyalkylenglykole, wie z.B. Polyethylenglykole oder Propylenglykole, Ester des Glycerins, wie z.B. Triacetin, Myvacet® (acetylierte Mono- und Diglyceride, $C_{23}H_{44}O_5$ bis $C_{25}H_{47}O_7$), mittelkettige Triglyceride (Miglyol®), Ölsäure oder Gemische aus wenigstens zwei der vorstehend genannten Welchmacher. Vorzugsweise enthalten wäßrige Dispersionen von Eudragit RS® und gegebenenfalls Eudragit RL® Triethylcitrat als Weichmacher.

Vorzugsweise enthält der retardierende Überzug den/die Weichmacher in Mengen von 5 bis 50 Gew-%., besonders bevorzugt 10 bis 40 Gew.-% und ganz besonders bevorzugt 10 bis 30 Gew-%, bezogen auf die Menge des eingesetzten Polymeren.

In Einzelfällen, beispielsweise für Celluloseacetat können auch höhere Mengen an Weichmachern, vorzugsweise bis zu 110 Gew.-%, bezogen auf die Menge Celluloseacetat, eingesetzt werden.

Desweiteren kann der retardierende Überzug weitere übliche, dem Fachmann bekannte Hilfsstoffe, wie z.B, Gleitmittel, vorzugsweise Talkum oder Glycerinmonostearat, Farbpigmente, vorzugsweise Eisenoxide oder Titandioxid, oder Tenside, wie z.B. Tween 80® aufweisen.

Das Freisetzungsprofil des retardierten Wirkstoffanteils kann durch die üblichen, dem Fachmann bekannten Methoden, wie z.B. durch die Dicke des Überzugs oder durch den Einsatz weiterer Hilfsstoffe als Bestandteile des Überzugs eingestellt werden. Geeignete Hilfsstoffe sind beispielsweise hydrophile oder pH-abhängige Porenbildner, wie z.B. Natrium-Carboxymethylcellulose, Celluloseacetatphthalat, Hydroxypropylmethylcelluloseacetatsuccinat, Lactose, Polyethylenglykol oder Mannitol oder wasserlösliche Polymere, wie z.B. Polyvinylpyrrolidon oder wasserlösliche Cellulosen, vorzugsweise Hydroxypropylmethylcellulose oder Hydroxypropylcellulose.

Der retardierende Überzug kann auch unlösliche bzw. lipophile Hilfsstoffe, wie z.B. alkylisiertes Siliciumdioxid, das z.B. als Aerosil R972® am Markt geführt wird, oder Magnesiumstearat zur weiteren Verstärkung der Retardierung enthalten.

Die jeweilige Formulierung des erfindungsgemäßen Arzneimittels kann ggf. neben dem retardierenden Überzug auch mindestens einen weiteren Überzug aufweisen. Dies kann beispielsweise ein Überzug zur Geschmacksverbesserung oder ein magensaftresistenter Überzug sein.

Der magensaftresistente Überzug basiert vorzugsweise auf Methacrylsäure/Methylmethacrylat-Copolymeren mit einem molaren Verhältnis der jeweiligen Monomeren von 1 :1 (Eudragit L®), Methacrylsäure/Methylmethacrylat-Copolymeren mit einem molaren Verhältnis der jeweiligen Monomeren von 1 : 2 (Eudragit S®), Methacrylsäure/Ethylacrylat-Copolymeren mit einem molaren Verhältnis der jeweiligen Monomeren von 1: 1 (Eudragit L30D-55®) Methacrylsäure/Methylacrylat/Methylmethacrylat-Copolymeren mit einem molaren Verhältnis der jeweiligen Monomeren von 7 : 3 : 1 (Eudragit FS®), Schellack Hydroxypropylmethylcellulosescetatsuccinat. Celluloseacetatphthalat oder einer Mischung aus wenigstens zwei dieser vorstehend genannten Komponenten, die ggf. auch in Kombination mit den vorstehend genannten wasserunlöslichen Poly(meth)acrylaten, vorzugsweise in Kombination mit Eudragit NE30D® und/oder Eudragit RL® und/oder Eudragit RS® eingesetzt werden können.

Die Überzüge können nach üblichen, für den jeweiligen Überzug geeigneten, dem Fachmann bekannten Verfahren, wie z.B. durch Aufsprühen von Lösungen, Dispersionen oder Suspensionen, durch Schmelzverfahren oder durch Pulverauftragsverfahren aufgebracht werden. Die Lösungen, Dispersionen oder Suspensionen können in Form von wäßrigen und/oder organischen Lösungen oder Dispersionen eingesetzt werden. Dabei werden wäßrige Dispersionen bevorzugt eingesetzt. Als organische Lösungsmittel können bevorzugt Alkohole, beispielsweise Ethanol oder Isopropanol, Ketone, wie z.B. Aceton, Ester, beispielsweise Ethylacetat, chlorierte Kohlenwasserstoffe, wie z.B. Dichlormethan verwendet werden, wobei Alkohole oder Ketone besonders bevorzugt eingesetzt werden. Es ist auch möglich Mischungen aus wenigstens zwei der vorstehend genannten Lösungsmittel einzusetzen.

Sofern das Arzneimittel In multipartikulärer Form vorliegt und der Wirkstoff zumindest teilweise retardiert abgegeben werden soll, wird der retardierende Oberzug vorzugsweise so aufgebracht, daß man die multipartikulären Formen enthaltend das Wirkstoffsalz nach ihrer Herstellung mit den entsprechenden Polymeren und ggf. einem anderen Wirkstoff und/oder demselben Wirkstoffsalz und gegebenenfalls weiteren physiologisch verträglichen Hilfsstoffen aus wässrigen und/oder organischen Medien, vorzugsweise aus wässrigen Medien, mit Hilfe des Wirbelschichtverfahrens überzieht und den Überzug vorzugsweise gleichzeitig bei üblichen Temperaturen in der Wirbelschicht trocknet und ggf. wenn nötig tempert.

Vorzugsweise erfolgt die Trocknung des Überzuges für Poly(meth)acrylatüberzüge bei einer Zulufttemperatur im Bereich von 30 bis 50 °C, besonders bevorzugt im Bereich von 35 bis 45 °C.

Für Überzüge auf Cellulosebasis, wie z.B. Ethylcellulose oder Celluloseacetat, erfolgt die Trocknung bevorzugt bei einer Temperatur im Bereich von 50 bis 80 °C, besonders bevorzugt im Bereich von 55 bis 65 °C.

Wachsüberzüge können durch Schmelzüberziehen in der Wirbelschicht aufgebracht werden und bei Temperaturen unterhalb des jeweiligen Schmelzbereiches nach dem Überziehen zur vollständigen Verfestigung abgekühlt werden. Das Aufbringen von Wachsüberzügen kann auch durch Aufsprühen von deren Lösungen in organischen Lösungsmitteln erfolgen.

Zur Modifizierung des Wirkstoff-Freisetzungsprofils kann das erfindungsgemäße Arzneimittel das zu retardierende pharmazeutische Salz auch in einer retardierenden Matrix, vorzugsweise gleichmäßig verteilt, enthalten.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether. Celluloseester und/oder Acrylharze verwendet. Ganz besonders bevorzugt werden als Matrixmaterialien Ethylcellulose. Hydroxypropytmethylcslluloae, Hydroxypropylcellulose, Hydroxymethylcellulose. Poly(meth)acrylsäure und/oder deren Derivate, wie z.B. deren Salze, Amide oder Ester eingesetzt.

Ebenfalls bevorzugt sind Matrixmateriallen aus hydrophoben Materialien, wie hydrophoben Polymeren, Wachsen, Fetten, langkettigen Fettsäuren, Fettalkoholen oder entsprechenden Estern oder Ethern oder Gemische aus wenigstens zwei der vorstehend genannten Materialien. Besonders bevorzugt werden als hydrophobe Materialien Mono- oder Diglyceride von $C_{12}$-$C_{30}$-Fettsäuren und/oder $C_{12}$-$C_{30}$Fettalkohole und/oder Wachse oder Gemische aus wenigstens zwei der vorstehend genannten Materialien eingesetzt.

Es ist auch möglich, Mischungen der vorstehend genannten hydrophilen und hydrophoben Materialien als retardierendes Matrixmaterial einzusetzen.

Die Herstellung der retardierenden Matrix kann nach den üblichen, dem Fachmann bekannten Methoden erfolgen.

Ein weiterer Gegenstand der Erfindung ist auch die Verwendung wenigstens eines erfindungsgemäßen pharmazeutischen Salzes und ggf. physiologisch verträglicher Hilfsstoffe zur Herstellung eines Arzneimittels. Die entsprechenden Arzneimittel können für die Behandlung der für die jeweiligen Wirkstoffe bekannten Indikationen verwendet werden.

Bevorzugt ist die Verwendung wenigstens eines erfindungsgemäßen pharmazeutisches Salzes zur Herstellung eines Arzneimittels zur Bekämpfung von Schmerzen, wobei als Salze bevorzugt die Saccharinate verwendet werden.

Ebenfalls bevorzugt ist die Verwendung wenigstens eines erfindungsgemäßen pharmazeutisches Salzes zur Herstellung eines Arzneimittels zur Behandlung von Harninkontinenz, wobei als Salze bevorzugt die Saccharinate verwendet werden.

Die an den Patienten zu verabreichende Gesemtmenge des jeweiligen pharmazeutischen Salzes variiert z.B. in Abhängigkeit vom Gewicht des Patienten, von der Indikation sowie dem Schweregrad der Schmerzen bzw. der Erkrankung. Dem Fachmann Ist aufgrund der Eigenschaften der jeweiligen Wirkstoffe bekannt, in welchen Dosierungen diese zu verabreichen sind, um die gewünschte Wirkung zu erzielen.

Die erfindungsgemäßen pharmazeutischen Salze aus einem pharmazeutischen Wirkstoff und einem Zuckerersatzstoff zeichnen sich gegenüber den herkömmlich verwendeten Salzen dieser Wirkstoffe üblicherweise durch eine geringere Löslichkeit in Wasser aus. Vorzugsweise sind dies die Saccharinate der jeweiligen Wirkstoffe, deren Wasserlöslichkeit meistens ≤ 250 mg/ml Wasser beträgt und gegenüber der Wasserlöslichkeit der herkömmlichen Salze des entsprechenden Wirkstoff meist um mindestens 50 %, verringert ist.

Hierdurch wird auch die Formulierung dieser pharmazeutischen Salze zu Arzneimitteln, beispielsweise die Herstellung von Granulaten durch Extrusion vereinfacht Die erfindungsgemäßen pharmazeutischen Salze ermöglichen ferner eine im Vergleich zu üblicherweise verwendeten Salzen auf Grund der geänderten Löslichkeit eine effektivere Retardierung des Wirkstoffes mit üblichen Retardierungsverfahren. Retardierte Arzneimittel, die diese erfindungsgemäßen pharmazeutischen Salze enthalten, können daher einfacher und kostengünstiger produziert werden. Dies gilt auch für andere Modifizierungen der erfindungsgemäßen Arzneimittel wie z. B. mit magensaftresistenten Überzügen.

Aus den erfindungsgemäßen Arzneimitteln, die zur Applikation des jeweiligen pharmazeutischen Salzes über die Mundschleimhaut oder den Darmtrakt eingesetzt werden, wird darüber hinaus eine weitgehend kontrollierte Freisetzung des jeweiligen Wirkstoffes ohne den Einsatz einer retardierenden Matrix und/oder eines retardierenden Überzuges, ggf. aber mit einem magensaftresistenten Überzug erreicht.

Die erfindungsgemäßen Arzneimittel in oral zu verabreichender Form, die den jeweiligen Wirkstoff bereits bei oder unmittelbar nach der Applikation freisetzen, haben weiterhin den Vorteil, daß Ihr stark bitterer oder ekelerregender Geschmack durch die gleichzeitige Freisetzung des Zuckerersatzstoffes kompensiert wird. Hierdurch verbessert sich die Einhaltung der Dosierungsvorschrift bei den Patienten und die Arzneimittel, die den jeweiligen Wirkstoff als Salz enthalten, erfahren eine größere Akzeptanz. Die erfindungsgemäßen Arzneimittel sind zudem auch für Diabetiker geeignet.

Für eine Vielzahl von Wirkstoffen ist die Wasserlöslichkeit der herkömmlichen Wirkstoffsalze bekannt, beispielsweise aus Pharmazeutische Stoffliste, 12. Auflage ABDATA Pharma-Daten-Service, 65735 Eschborn/Taunus.

Sofern die Wasserlöslichkeit eines Wirkstoffsalzes nicht bekannt ist, kann sie gemäß der nachstehend angegebenen Methode bestimmt werden, nach der auch die Wasserlöslichkeit der erfindungsgemäßen pharmazeutischen Salze bestimmt wurde:

In einem klaren farblosen Gefäß aus durchsichtigem Material, wie beispielsweise Glas oder Kunststoff wird bei einer Temperatur von 20 °C 1 ml Ionenfreies Wasser oder ein Bruchteil (Menge A in ml) davon vorgelegt. Unter Rühren mit einem Magnetrührstab wurde dann das zu prüfende herkömmliche Wirkstoffsalz bzw. das erfindungsgemäße pharmazeutische Salz portionsweise zugegeben.

Sofern sich die zugegebene Salzmenge B (in mg) vollständig aufgelöst hat, wurden langsam weitere Mengen des jeweiligen Salzes zugegeben. Jede weitere Zugabe wurde dokumentiert und das Lösungsverhalten beobachtet. Sobald die erste Trübung durch ungelöstes Salz durch Beobachtung gegen einen geeigneten Hintergrund festgestellt wurde, wurde weitere 10 Minuten nachgerührt. Sofern anschließend ungelöste Bestandteile zurückblieben, wurde die Summe C (in mg) der eingesetzten Menge an Substanz bestimmt. Entstand beim Rühren wieder eine klare Lösung, wurden solange weitere geringe Mengen des jeweiligen Salzes zugegeben und Jeweils erneut für 10 Minuten gerührt, bis eine erste Trübung aufgrund von ungelöstem Salz zurückblieb. Anschließend wurde die überschüssige Menge an ungelöster Substanz durch Zugabe von geringen Mengen Wasser unter Rühren in Lösung gebracht. Nach Erhalt einer klaren Lösung wurde die Summe D (in ml) der eingesetzten Wassermenge ermittelt. Die Löslichkeit des jeweiligen Salzes pro 1 ml Wasser wurde dann nach folgender Formel berechnet:

$$\text{Wasserlöslichkeit des Wirkstoffsalzes in mg/ml Wasser} = \frac{(C/A) + (C/D)}{2}$$

Sofern sich die zugegebene Menge B (in mg) des jeweiligen Salzes nicht sofort aufgelöst hat und eine Trübung entstand, wurde nach der Zugabe des Salzes weitere 10 Minuten lang gerührt. Blieb anschließend immer noch ungelöstes Salz zurück, wurde der nicht gelöste Anteil durch Zugabe von geringen Wassermengen unter Rühren in Lösung gebracht Nach Erhalt einer klaren Lösung wurde die Summe E (in ml) der eingesetzten Wassermengen ermittelt. Die Löslichkeit des jeweiligen Salzes pro 1 ml Wasser wurde dann nach folgender Formel berechnet:

$$\text{Wasserlöslichkeit des Wirkstoffsalzes in mg/ml Wasser} = \frac{B}{E}$$

Im folgenden wird die Erfindung anhand von Beispielen erläutert.

**Beispiele:**

Beispiel 1:

Die Herstellung und die anschießende Abtrennung der optisch reinen Verbindung (+)-(1 S,2S)-3-(3-Dimethylamino-1-ethyl-2-methyl-propyl)-phenol erfolgte gemäß der DE-A-4426245. Der entsprechende Teil der Offenbarung wird hiermit als Referenz eingeführt und gilt somit als Teil der Offenbarung.

Zur Herstellung von (+)-(1S,2S)-3-(3-Dimethylamino-1-ethyl-2-methylpropyl)-phenolsaccharinat wurden 2,58 g (10 mmol) (+)-(1S,2S)-3-(3-Dimethylamino-1-ethyl 2-methyl-propyl)-phenolhydrochlorid und 2,42 g (10 mmol) Saccharin-Natrium-Dihydrat jeweils unter Erwärmen in einer möglichst geringen Menge Wasser vollständig gelöst. Anschließend wurden beide Lösungen unter Rühren miteinander vermischt und dann Ober Nacht kühl gestellt. Das ausgefallene (+)-(1S,2S)-3-(3-Dimethylamino-1-ethyl-2-methyl-propyl)-phenolsaccharinat wurde von der überstehenden Mutterlauge abgetrennt, mit Ethanol gereinigt und nach herkömmlichen Methoden isoliert.

REFERENZ-Beispiel 2:

Zur Herstellung von Diphenhydraminsaccharinat wurden 5,0 g (17,1 mmol) Diphenhydraminhydrochlorid und 4,13 g (17,1 mmol) Saccharin-Natrium-Dihydrat jeweils unter Erwärmen in einer möglichst geringen Menge Wasser vollständig gelöst Anschließend wurden beide Lösungen unter Rühren miteinander vermischt und dann über Nacht kühl gestellt. Das ausgefallene Diphenhydraminsaccharinat wurde von der überstehenden Mutterlauge abgetrennt, mit Ethanol gereinigt und nach herkömmlichen Methoden isoliert.

REFERENZ-Beispiel 3:

Zur Herstellung von Verapamllsaccharinat wurden 415 mg (0,845 mmol) Verapemilhydrochlorid und 204 mg (0,845 mmol) Saccharin-Natrium-Dihydrat jeweils unter Erwärmen in einer möglichst geringen Menge Wasser vollständig gelöst Anschließend wurden beide Lösungen unter Rühren miteinander vermischt und dann über Nacht kühl gestellt. Das ausgefallene Verapamilsaccharinat wurde von der überstehenden Mutterlauge abgetrennt, mit Ethanol gereinigt und nach herkömmlichen Methoden isoliert.

REFERENZ-Beispiel 4:

Zur Herstellung von Morphinsaccharinat wurden 285 mg (0.76 mmol) Morphinhydrochlorid-Trihydrat und 183 mg (0,76 mmol) Saccharin-Natrium-Dihydrat jeweils unter Erwärmen in einer möglichst geringen Menge Wasser vollständig gelöst. Anschließend wurden beide Lösungen unter Rühren miteinander vermischt und dann über Nacht kühl gestellt. Das ausgefallene Morphinsaccharinat wurde von der überstehenden Mutterlauge abgetrennt, mit Ethanol gereinigt und nach herkömmlichen Methoden isoliert.

REFERENZ-Beispiel 5:

Zur Herstellung eines oralen Gels wurden zunächst 0,33 g Methylparaben, 0,05 g Propylparaben und 75,0 g Xylitol bei einer Temperatur von 80 °C in 198,0 g gereinigtem Wasser gelöst und die Mischung anschließend auf 40 °C abgekühlt. Dann wurden unter Rühren zunächst 0,94 g gemäß REFERENZ-Beispiel 2 erhaltenes Diphenhydraminsaccharinat und anschließend 2 g Xanthangummi zugegeben, eine Stunde nachgerührt und verdunstetes Wasser ersetzt. Nach dem Abkühlen auf eine Temperatur von 20 bis 25 °C wurde die Mischung unter Rühren mit 0,625 g Tutti Frutti 9/008897 (Dragoco Gerberding & Co. AG, 37603 Holzminden) aromatisiert.

REFERENZ-Beispiel 6:

5 g zerkleinerte Kaugummimasse (Popeye Amural Confections, Yorkville, Illinois. USA) wurden in einer Fantaschale auf eine Temperatur von 30 bis 40 °C erwärmt In die zähflüssige Kaugummimasse wurden dann mit einem Pistill 187,5 mg gemäß REFERENZ-Beispiel 2 erhaltenes Diphenhydraminsaccharinat eingearbeitet. Die homogene Masse wurde dann in teflonisierten Formen zu Portionen von je 1g portioniert.

Der Geschmackstest ergab, daß die Kaugummis, die das Diphenhydraminsaccharinat enthielten, zu Anfang einen ausgezeichneten Geschmack aufwiesen und auch nach längerer Kauzeit noch genießbar waren.

Beispiel 7:

Zur Herstellung eines Saftes auf wäßriger Grundlage wurden wurden 0,33 g Methylparaben, 0,05 g Propylparaben und 75,0 g Xylitol bei einer Temperatur von 80 °C in 199,22 g gereinigtem Wasser gelöst Die Mischung wurde auf 40 °C abgekühlt und unter Rühren wurden 78,5 mg gemäß Beispiel 1 erhaltenes (+)-(1S,2S)-3-(3-Dimethylamino-1-ethyl-2-methyl-propyl)-phenolsaccharinat zugegeben. Anschließend wurden 0,25 g Xanthangummi zugegeben, eine Stunde nachgerührt und verdunstetes Wasser ersetzt Nach dem Abkühlen auf Temperatur von 20 bis 26 °C wurde die Mischung unter Rühren mit 0,075 g Orange-Mandarine Flavor 10688-56 (Glvaudan Roure Flavors Ltd. CH 8600 Dübendorf) aromatisiert.

Beispiel 8:

In diesem Beispiel wurde die Wasserlöslichkeit von bestimmten pharmazeutischen Salzen sowie von herkömmlichen Salzen des entsprechenden Wirkstoffs nach der obenstehend angegebenen Methode bestimmt. Die so erhaltenen Löslichkeitswerte sind in der nachfolgenden Tabelle 1 widergegeben;

**Tabelle 1:**

| Vergleich der Wasserlöslichkeiten von bestimmten erfindungsgemäßen pharmazeutischen Salzen und entsprechenden herkömmlichen Salzen dieser Wirkstoffe. Das jeweils eingesetzte herkömmliche Salz ist In Klammem angegeben. | | |
|---|---|---|
| Wirkstoff | Löslichkeit des Wirkstoffsalzes in mg/ml Wasser | Löslichkeit des Wirkstoffsacdiarinats in mg/ml Wasser |
| (-)-(1R,2R)-3-(3-Dimethylamino-1-ethyl-2-methyl-propyl)phenol | 261 (Hydrochlorid) | 31 |
| (1RS,3RS,6RS)-6-Dimethylaminomethyl)-1-(3-methoxy-phenyl)-cyclohexan-1,3-diol [1] | 500 (Hydrochlorid) | 71 |
| (+)-(1S,2S)-3-(3-Dimethylamino-1-ethyl-2-methyl-propyl)-phenol | 650 (Hydrochlorid) | 55 |
| (-)-(1S,2S)-3-(3-Dimethylamino-1-ethyl-1-fluoro-2-methyl-propyl)-phenol | 568 (Hydrochlorid) | 130 |
| (-)-(2S,3S)-1-Dimethylamino-3-(3-methoxy-phenyl)-2-methyl-pentan-3-ol | 2000 (Hydrochlorid) | 90 |
| (+)-(1R,2R,4S)-2-Dimethylaminomethyl-4-(4-fluoro-benzyloxy)-1-(3-methoxyphenyl)-cyclohexanol [1] | 33 (Hydrochlorid) | 10 |
| Morphin [1] | 52 (Hydrochlorid-Trihydrat) | 25 |
| Amezinium [1] | 25 (metilsulfat) | 8 |
| Phenylephrin [1] | 1250 (Hydrochlorid) | 380 |
| Verapamil [1] | 200 (Hydrochlorid) | 7 |
| Diphenhydramin [1] | 1000 (Hydrochlorid) | 7 |
| Benzalkonium [1] | 500 (Hydrochlorid) | <2 |
| Codein [1] | 250 (Phosphat-hemihydrat) | 200 |
| Hydromorphon [1] | 330 (Hydrochlorid) | 130 |
| Buprenorphin [1] | 14 (Hydrochlorid) | 2 |
| [1] REFERENZ-BEISPIELE | | |

Wie aus den Löslichkeitswerten gemäß Tabelle 1 hervorgeht, Ist die Löslichkeit der jeweiligen Wirkstoffsaccharinate gegenüber den entsprechenden herkömmlichen Wirkstoffsalzen verringert.

**Patentansprüche**

1. Pharmazeutisches Salz aus einem pharmazeutischen Wirkstoff und wenigstens einem Zuckerersatzstoff, **dadurch gekennzeichnet, daß** der Wirkstoff eine salzbildende Verbindung ist ausgewählt aus der Gruppe bestehend aus

   (1RS,2RS)-3-(3-Dimethylamino-1-hydroxy-1,2-dimethyl-propyl)phenol,
   (-)-(1R,2R)-3-(3-Dimethylamino-1-ethyl-2-methyl-propyl)-phenol,
   (+)-(1S,2S)-3-(3-Dimethylamino-1-ethyl-2-methyl-propyl)-phenol
   (2RS,3RS)-1-Dimethylamino-3-(3-methoxy-phenyl)-2-methyl-pentan-3-ol,
   (-)-(1S,2S)-3-(3-Dimethylamino-1-ethyl-1-fluoro-2-methyl-propyl)-phenol,
   (+)-(1R,2R)-3-(3-Dimethylamino-1-hydroxy-1,2-dimethyl-propyl)-phenol,
   (+)-(2R,3R)-1-Dimethylamino-3-(3-methoxy-phenyl)-2-methyl-pentan-3-ol und
   (-)-(2S,3S)-1-Dimethylamino-3-(3-methoxy-phenyl)-2-methyl-pentan-3,oi.

2. Pharmazeutisches Salz nach Anspruch 1, **dadurch gekennzeichnet, daß** die Löslichkeit des Salzes in Wasser $\leq$ 250 mg/ml Wasser, vorzugsweise $\leq$ 200 mg/ml, besonders bevorzugt $\leq$ 150 mg/ml, ganz besonders bevorzugt $\leq$ 100 mg/ml beträgt.

3. Pharmazeutisches Salz nach Anspruch 1 oder 2, **dadurch gekennzeichnet, daß** der salzbildende Zuckerersatzstoff Saccharin, Cyclamat oder Acesulfam, vorzugsweise Saccharin ist.

4. Arzneimittel enthaltend wenigstens ein pharmazeutisches Salz nach einem der Ansprüche 1 bis 3 und gegebenenfalls physiologisch verträgliche Hilfsstoffe.

5. Arzneimittel enthaltend wenigstens ein pharmazeutisches Salz nach einem der Ansprüche 1 bis 3 zur Bekämpfung von Schmerzen.

6. Arzneimittel enthaltend wenigstens ein pharmazeutisches Salz nach einem der Ansprüche 1 bis 3 zur Behandlung von Harninkontinenz.

7. Arzneimittel nach einem der Ansprüche 4 bis 6, **dadurch gekennzeichnet, daß** es in Form von Gelen, Kaugummis, Säften, Sprays, Tabletten, Kautabletten, Dragees, Pulver ggf. in Kapseln abgefüllt, leicht rekonstituierbaren Trokkenzubereitungen, vorzugsweise in Form von Gelen, wäßrigen oder öligen Säften, Sublingualsprays, Tabletten oder Kautabletten formuliert vorliegt.

8. Arzneimittel nach einem der Ansprüche 4 bis 6, **dadurch gekennzeichnet, daß** es in multipartikulärer Form, vorzugsweise in Form von Mikrotabletten, Mikrokapseln, Granulaten, Wirkstoffkristallen oder Pellets, besonders bevorzugt in Form von Mikrotabletten, Granulaten oder Pellets, ggf. in Kapseln abgefüllt oder zu Tabletten verpreßt, formuliert vorliegt.

9. Arzneimittel nach einem der Ansprüche 4 bis 8, **dadurch gekennzeichnet, daß** das Salz zumindest teilweise in retardierter Form vorliegt.

10. Arzneimittel nach Anspruch 9, **dadurch gekennzeichnet, daß** die Retardierung durch Aufbringen eines retardierenden Überzugs, Einbettung in eine retardierende Matrix, Anbindung an ein Ionenaustauscherharz oder durch eine Kombination von wenigstens zwei dieser Methoden erfolgt ist.

11. Arzneimittel nach Anspruch 10, **dadurch gekennzeichnet, daß** der retardierende Überzug auf einem wasserunlöslichen ggf. modifizierten natürlichen oder synthetischen Polymer ggf. in Kombination mit einem üblichen Weichmacher oder auf einem natürlichen, halbsynthetischen oder synthetischen Wachs oder Fett oder Fettalkohol oder einem Gemisch aus wenigstens zwei dieser Komponenten basiert.

12. Arzneimittel nach Anspruch 10, **dadurch gekennzeichnet, daß** die Matrix auf einem hydrophilen Matrixmaterial, vorzugsweise hydrophilen Polymeren, besonders bevorzugt auf Celluloseethern, Celluloseestern und/oder Acrylharzen, ganz besonders bevorzugt auf Ethylcellulose, Hydroxypropylmethylcellulose, Hydroxypropylcellulose, Hydroxymethylcellulose, Poly(meth)acrylsäure und/oder deren Salzen, Amiden und/oder Estern basiert.

13. Arzneimittel nach Anspruch 10, **dadurch gekennzeichnet, daß** die Matrix auf einem hydrophoben Matrixmaterial, vorzugsweise hydrophoben Polymeren, Wachsen, Fetten, langkettigen Fettsäuren, Fettalkoholen oder entsprechenden Estern oder Ethern oder deren Gemischen, besonders bevorzugt auf Mono- oder Diglyceriden von $C_{12}$-$C_{30}$-Fettsäuren und/oder $C_{12}$-$C_{30}$-Fettalkoholen und/oder Wachsen oder deren Gemischen basiert.

14. Arzneimittel nach einem der Ansprüche 4 bis 13, **dadurch gekennzeichnet, daß** es einen Schutzüberzug, vorzugsweise einen magensaftresistenten Schutzüberzug aufweist.

15. Verwendung wenigstens eines pharmazeutischen Salzes nach einem der Ansprüche 1 bis 3 zur Herstellung eines Arzneimittels zur Bekämpfung von Schmerzen.

16. Verwendung wenigstens eines pharmazeutischen Salzes nach einem der Ansprüche 1 bis 3 zur Herstellung eines Arzneimittels zur Behandlung von Harninkontinenz.

**Claims**

1. A pharmaceutical salt of a pharmaceutical active compound and at least one sugar substitute **characterized in that** the active compound is a salt-forming compound selected from the group consisting of

   (1RS,2RS)-3-(3-dimethylamino-1-hydroxy-1,2-dimethylpropyl)phenol,
   (-)-(1R,2R)-3-(3-dimethylamino-1-ethyl-2-methylpropyl)phenol,
   (+)-(1S,2S)-3-(3-dimethylamino-1-ethyl-2-methylpropyl)phenol,
   (2RS,3RS)-1-dimethylamino-3-(3-methoxyphenyl)-2-methylpentan-3-ol,
   (-)-(1S,2S)-3-(3-dimethylamino-1-ethyl-1-fluoro-2-methylpropyl)phenol,
   (+)-(1R,2R)-3-(3-dimethylamino-1-hydroxy-1,2-dimethylpropyl)phenol,
   (+)-(2R,3R)-1-dimethylamino-3-(3-methoxyphenyl)-2-methylpentan-3-ol and
   (-)-(2S,3S)-1-dimethylamino-3-(3-methoxyphenyl)-2-methylpentan-3-ol.

2. The pharmaceutical salt as claimed in claim 1, **characterized in that** the solubility of the salt in water is $\leq 250$ mg/ml of water, preferably $\leq 200$ mg/ml, particularly preferably $\leq 150$ mg/ml, very particularly preferably $\leq 100$ mg/ml.

3. The pharmaceutical salt as claimed in claim 1 or 2, **characterized in that** the salt-forming sugar substitute is saccharin, cyclamate or acesulfam, preferably saccharin.

4. Medicament comprising at least one pharmaceutical salt as claimed in one of Claims 1 to 3 and, if appropriate, physiologically tolerable excipients.

5. Medicament comprising at least one pharmaceutical salt as claimed in one of Claims 1 to 3 for the control of pain.

6. Medicament comprising at least one pharmaceutical salt as claimed in one of Claims 1 to 3 for the control of urinary incontinence.

7. Medicament as claimed in one of Claims 4 to 6, **characterized in that** it is present formulated in the form of gels, chewing gums, juices, sprays, tablets, chewable tablets, coated tablets, powders, if appropriate filled into capsules, easily reconstitutable dry preparations, preferably in the form of gels, aqueous or oily juices, sublingual sprays, tablets or chewable tablets.

8. Medicament as claimed in one of Claims 4 to 6, **characterized in that** it is present formulated in multiparticulate form, preferably in the form of microtablets, microcapsules, granules, active compound crystals or pellets, particularly preferably in the form of microtablets, granules or pellets, optionally filled into capsules or compressed to give tablets.

9. Medicament as claimed in one of Claims 4 to 8, **characterized in that** the salt is present at least partially in delayed-release form.

10. Medicament as claimed in Claim 9, **characterized in that** delaying of the release is carried out by applying a release-delaying coating, embedding in a release-delaying matrix, binding to an ionexchange resin or by a combination of at least two of these methods.

**11.** Medicament as claimed in Claim 10, **characterized in that** the release-delaying coating is based on a water-insoluble, optionally modified natural or synthetic polymer, optionally in combination with a customary plasticizer, or on a natural, semisynthetic or synthetic wax or fat or fatty alcohol or Mixture of at least two of these components.

**12.** Medicament as claimed in Claim 10, **characterized in that** Matrix is based on a hydrophilic matrix material, preferably hydrophilic polymers, particularly preferably on cellulose ethers, cellulose esters and/or acrylic resins, very particularly preferably on ethylcellulose, hydroxypropylmethylcellulose, hydroxypropylcellulose, hydroxymethylcellulose, poly(meth)acrylic acid and/or their their salts, amides and/or esters.

**13.** Medicament as claimed in Claim 10, **characterized in that** Matrix is based on a hydrophobic matrix material, preferably hydrophobic polymers, waxes, fats, long-chain fatty acids, fatty alcohols or appropriate esters or ethers or their mixtures, particularly preferably on mono- or diglycerides of $C_{12}$-$C_{30}$ fatty acids and/or $C_{12}$-$C_{30}$-fatty alcohols and/or waxes or their mixtures.

**14.** Medicament as claimed in one of Claims 4 to 13, **characterized in that** it has a protective coating, preferably an enteric protective coating.

**15.** Use of at least one pharmaceutical salt as claimed in one of Claims 1 to 3 for the production of Medicament for the control of pain.

**16.** Use of at least one pharmaceutical salt as claimed in one of Claims 1 to 3 for the production of Medicament for the treatment of urinary incontinence.

**Revendications**

**1.** Sel pharmaceutique d'une substance active pharmaceutique et d'au moins un produit de remplacement du sucre, **caractérisé en ce que** la substance active est un composé formant un sel, choisi dans le groupe formé par (1RS, 2RS)-3-(3-diméthylamino-1-hydroxy-1,2-diméthylpropyl)phénol, (-)-(1R,2R)-3-(3-diméthylamino-1-éthyl-2-méthylpropyl)-phénol, (+)-(1S,2S)-3-(3-diméthylamino-1-éthyl-2-méthylpropyl)phénol, (2RS,3RS)-1-diméthylamino-3-(3-méthoxyphényl)-2-méthylpentan-3-ol, (-)-(1S,2S)-3-(3-diméthylamino-1-éthyl-1-fluoro-2-méthylpropyl)-phénol, (+)-(1R,2R)-3-(3-diméthylamino-1-hydroxy-1,2-diméthylpropyl)phénol, (+)-(2R,3R)-1-diméthylamino-3-(3-méthoxyphényl)-2-méthylpentan-3-ol et (-)-(2S,3S)-1-diméthylamino-3-(3-méthoxyphényl)-2-méthylpentan-3-ol.

**2.** Sel pharmaceutique selon la revendication 1, **caractérisé en ce que** la solubilité du sel dans l'eau est $\leq$ 250 mg/ml d'eau, de préférence $\leq$ 200 mg/ml, de manière particulièrement préférée $\leq$ 150 mg/ml, de manière tout particuliè-rement préférée $\leq$ 100 mg/ml.

**3.** Sel pharmaceutique selon la revendication 1 ou 2, **caractérisé en ce que** le produit de remplacement du sucre formant un sel est la saccharine, le cyclamate ou l'acésulfame, de préférence la saccharine.

**4.** Médicament contenant au moins un sel pharmaceutique selon l'une quelconque des revendications 1 à 3 et le cas échéant des adjuvants physiologiquement acceptables.

**5.** Médicament contenant au moins un sel pharmaceutique selon l'une quelconque des revendications 1 à 3 pour lutter contre les douleurs.

**6.** Médicament contenant au moins un sel pharmaceutique selon l'une quelconque des revendications 1 à 3 pour le traitement de l'incontinence urinaire.

**7.** Médicament selon l'une quelconque des revendications 4 à 6, **caractérisé en ce qu'**il est formulé sous forme de gels, de gommes à mâcher, de jus, de sprays, de comprimés, de comprimés à mâcher, de dragées, de poudres, le cas échéant transvasées dans des capsules, de préparations sèches facilement reconstituables, de préférence sous forme de gels, de jus aqueux ou huileux, de sprays sublinguaux, de comprimés ou de comprimés à mâcher.

**8.** Médicament selon l'une quelconque des revendications 4 à 6, **caractérisé en ce qu'**il est formulé sous forme multiparticulaire, de préférence sous forme de microcomprimés, de microcapsules, de granulés, de cristaux de substances actives ou de pellets, de manière particulièrement préférée sous forme de microcomprimés, de granulés ou de pellets, le cas échéant transvasé dans des capsules ou pressé en comprimés.

9. Médicament selon l'une quelconque des revendications 4 à 8, **caractérisé en ce que** le sel se trouve au moins partiellement sous une forme retardée.

10. Médicament selon la revendication 9, **caractérisé en ce que** le retard est réalisé par l'application d'un revêtement à effet de retard, l'incorporation dans une matrice à effet de retard, la fixation sur une résine échangeuse d'ions ou par une combinaison d'au moins deux de ces procédés.

11. Médicament selon la revendication 10, **caractérisé en ce que** le revêtement à effet de retard est à base d'un polymère naturel ou synthétique, insoluble dans l'eau, le cas échéant modifié, le cas échéant en combinaison avec un plastifiant usuel ou à base d'une cire ou d'une graisse ou d'un alcool gras naturel, semi-synthétique ou synthétique ou d'un mélange d'au moins deux de ces composants.

12. Médicament selon la revendication 10, **caractérisé en ce que** la matrice est à base d'un matériau de type matrice hydrophile, de préférence de polymères hydrophiles, de manière particulièrement préférée à base d'éthers de cellulose, d'esters de cellulose et/ou de résines acryliques, de manière tout particulièrement préférée à base d'éthylcellulose, d'hydroxypropylméthylcellulose, d'hydroxypropylcellulose, d'hydroxyméthylcellulose, de poly(acide (méth)acrylique) et/ou de leurs sels, amides et/ou esters.

13. Médicament selon la revendication 10, **caractérisé en ce que** la matrice est à base d'un matériau de type matrice hydrophobe, de préférence de polymères hydrophobes, de cires, de graisses, d'acides gras à longue chaîne, d'alcools gras ou d'esters ou d'éthers correspondants ou de leurs mélanges, de manière particulièrement préférée à base de monoglycérides ou de diglycérides d'acides gras en $C_{12}$-$C_{3o}$ et/ou d' alcools gras en $C_{12}$-$C_{30}$ et/ou de cires ou de leurs mélanges.

14. Médicament selon l'une quelconque des revendications 4 à 13, **caractérisé en ce qu'**il présente un revêtement de protection, de préférence un revêtement de protection résistant au suc gastrique.

15. Utilisation d'au moins un sel pharmaceutique selon l'une quelconque des revendications 1 à 3 pour la préparation d'un médicament destiné à lutter contre les douleurs.

16. Utilisation d'au moins un sel pharmaceutique selon l'une quelconque des revendications 1 à 3 pour la préparation d'un médicament destiné au traitement de l'incontinence urinaire.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4426245 A